# EUROPEAN PATENT APPLICATION

(11) **EP 1 186 228 A1**
(43) Date of publication of application: **13.03.2002**
(21) Application number: 00937326.7
(22) Date of filing: 20.06.2000
(51) Int. Cl.: A01H 4/00

(54) **METHOD FOR CONSTRUCTING VIRUS-FREE PLANT**

(30) Priority: 22.06.1999 JP 17576899
(71) Applicant: WAKUNAGA PHARMACEUTICAL CO., LTD., Osaka-shi, Osaka 532-0003 (JP)
(72) Inventor: AYABE, Masanori, Takata-gun, Hiroshima 739-1105 (JP); SUMI, Shinichiro, Takata-gun, Hiroshima 739-1105 (JP)
(74) Representative: Wibbelmann, Jobst, Dr., Dipl.-Chem.
(86) International application number: JP0004022
(87) International publication number: WO0078128

(57) **Abstract**

It is expected that the domy tissue formed by culturing the foliage leaf base in a scaly bulb or bulb is undergoing active cell division and is virus-free. The domy tissue may be isolated and cultured to obtain virus-free plants.

## Description

### Technical Field

The present invention relates to methods of generating virus-free plants of the genus *Allium* represented by *Allium sativum* and other plants that propagate via scaly bulbs and bulbs. More specifically, the present invention relates to methods of generating virus-free plants by isolating and culturing domy tissues obtained by culturing foliage leaf bases.

### Background Art

Plants belonging to the genus *Allium* include garlic, onion, scallion, cibol, shallot, leek, chive and the like. They are widely used as edible plants and spice. Since ancient days garlic has been recognized to have medicinal values, and is also used as aphrodisiac, antasthenic, and the like. Plants of the genus *Allium* have been cultured in many parts of the world including Japan. In recent years, however, damages by viruses are causing serious problems in their culturing. As such viruses for *Allium sativum,* for example, there are known garlic viruses (GarVs), leek yellow stripe virus (LYSV), onion yellow dwarf virus (OYDV), garlic latent virus (GLV) and the like. It is known that garlic is a crop that grows through vegetative propagation and thus, once it is infected with a virus, the infection is inherited to later generations resulting in the spread of viral pollution. Similar damage is known for plants belonging to the genus *Allium* other than garlic, and there is an urgent need for essential measures to combat such viral pollutions.

On the other hand, tissue culture methods for plants were recently established, the application of which method allows elimination of viruses for various plants that grow via vegetative propagation. It is generally known that even virus-infected plants have no viruses present in the shoot apex (meristem). Thus, by culturing the shoot apex and thereby redifferentiating the plants, plants that are not infected with virus (virus-free plants) can be obtained. It is also known that by culturing a plant tissue to form a callus and then subculturing the callus, virus can be eliminated. By redifferentiating the plant from the virus-free callus, virus-free plants can be obtained.

For plants of the genus *Allium* represented by *Allium sativum,* the above method has been employed to eliminate viruses, and in fact virus-free *Allium* plants have been cultivated.

However, by the method of culturing the shoot apex to obtain virus-free *Allium* plants, one shoot apex usually produces only one or a few plants. In order to obtain a multiplicity of plants, many shoot apexes must be extracted. Furthermore, since the shoot apex is located at the base of *Allium* ramentum and its size is about 0.5 mm or less, the extraction of the shoot apex requires an operation under the microscope. Thus, the operation of extracting shoot apexes is troublesome and the work efficiency is low.

On the other hand, in the method of redifferentiating the callus to obtain virus-free *Allium* plants, it is possible to grow the callus in large quantities once the callus has been induced. However, due to mutations frequently encountered during callus cultivation, it is difficult to obtain *Allium* plants of homogeneous genetic traits.

Accordingly, a tissue culture method for culturing in large quantities an *Allium* plant that was rendered virus-free by the shoot apex culture and a method of preparing redifferentiated plants using the base of foliage leaves were developed, which made possible to grow a large quantity of virus-free plants starting with a small amount thereof as the material (Japanese Unexamined Patent Publication (Kokai) No. 6-197650). However, these methods require, at all times, virus-free plants that are used as the material, which in turn requires maintaining said plants in an isolated cultivation using a net house.

### Disclosure of the Invention

The present invention relates to a culture method for generating virus-free plants from a tissue other than the shoot apex based on the plants infected with virus.

By culturing the base of foliage leaves, plant differentiation can be induced via a domy tissue. The inventors of the present invention have found for the first time that the domy tissue is undergoing active cell division and no virus is present therein, as in the shoot apex. Thus, by isolating only the domy tissue and culturing it, it has become possible to generate virus-free plants. Since a plurality of domy tissues are formed from one ramentum, the efficiency becomes dramatically enhanced as compared to the conventional method of shoot apex culture.

Thus, the present invention provides a method of generating virus-free plants characterized in that a domy tissue formed by culturing an explant comprising the foliage leaf base of a plant that propagates via scaly bulbs or bulbs is isolated and cultured.

Preferably, the explant is a foliage leaf base from which the shoot apex'and the foliage leaf have been removed, and the explant is cultured in the absence of plant hormones to form a domy tissue.

Preferably, the foliage leaf base is a section from the joint to a part 1-3 mm lower therefrom of a foliage leaf.

The plants that propagate by scaly bulbs or bulbs are preferably the plants of the genus *Allium.*

Said plants of the genus *Allium* are preferably *Allium sativum*.

### Brief Description of the Drawing

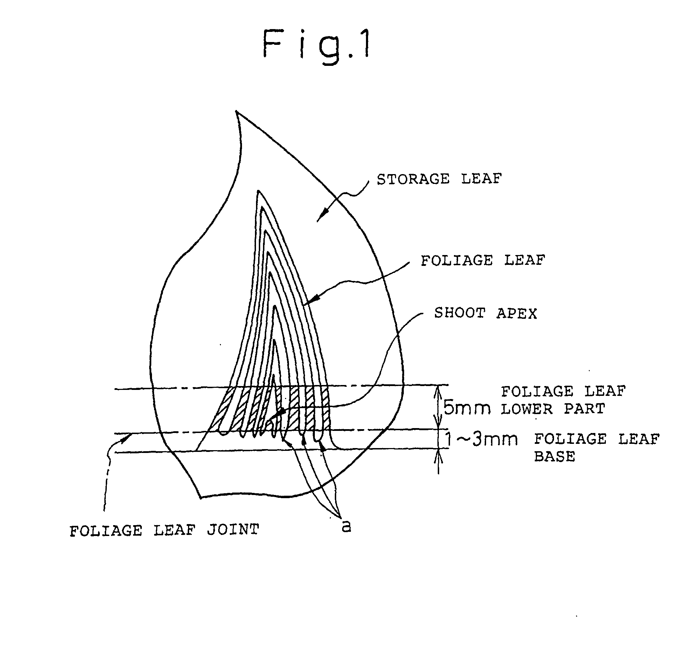

Figure 1 is a schematic cross-sectional view of the scaly bulb of *Alllum sativum.*

### Best Mode for Carrying Out the Invention

### (a) Applicable plants

The present invention applies to plants that form scaly bulbs having foliage leaves or bulbs. Such plants include lilies, narcissuses, tulips and the like, and most preferably plants of the genus *Allium* especially *Allium sativum.*

### (b) Preparation of explants

As an explant, the present invention uses the base of a foliage leaf. Methods of extracting a foliage leaf base from a scaly bulb or a bulbs involves, for example, the sterilizing scaly bulbs or bulbs that were cut into a suitable size with a bacteriocide that has no direct effect on plant cells such as sodium hypochlorite, benzalkonium chloride, ethyl alcohol etc., washing then adequately with a sterilized water, removing the stored leaves thereby to expose the foliage leaf base, and after removing the upper part of the foliage leaf and the shoot apex, excising the base of the remaining the foliage leaf into a suitable size, for example about 1-3 mm, which is subjected to culturing (Japanese Unexamined Patent Publication (Kokai) No. 6-197650). The position of the foliage leaf base in the scaly bulb of garlic is shown in Figure 1.

### (c) Culture medium used

As the culture media, any medium that contains inredients that are essential for plant growth including inorganic salts, organic salts such as vitamins, carbon sources, regulating agents of plant growth, and the like can be used. In accordance with the present invention, Murashige and Skoog medium, Linsmaier and Skoog medium and the like can be used.

### (d) Culture

An explant prepared as described in (b) is implanted into the above medium, and is cultured at a temperature (10-30°C, preferably 20-26°C) suitable for plant growth at an illumination of 50-15000 lux, preferably 3000-8000 lux (9-18 hours daily, preferably 12-16 hours), which forms a domy tissue in 5-7 days. When culture is continued, this domy tissue grows into a plant. In this culture method, the domy tissue is isolated from the explant and cultured. Domy tissues formed in 5-7 days of culture are excised with a razor blade or a scalpel, and they are again implanted in the medium shown in (c). By culturing in a similar manner, the domy tissues turn green and grow into small plantlets in about a month. When culture is further continued, the plantlets become rooted.

### (e) Cultivation

The rooted plantlets obtained in the above culture (d) are implanted into a polypot containing a suitable potting compost and grown to produce seedlings. The grown plantlets are implanted into a larger pot or into the filed for cultivation.

### (f) Virus testing

The plant obtained in the main cultivation is subjected to virus testing to insure that the plant is virus-free. The virus testing can be performed by a method established for each plant. For *Allium* plants, for example, there is a testing method that uses antivirus antibody or a testing method for confirming the presence or absence of a viral gene by the PCR method (PHYTOPATHOLOGY, Vol. 86, No. 3, 253-259 (1996)).

### Example

This example shows a culture method for generating virus-free plants using *Allium sativum* L. among plants of the genus *Allium.*

As a material for culturing, Fukuchi white sp. and *Allium sativum* L. native to Hokkaido were used. The strains of Fukuchi white sp. used were infected with one virus, leek yellow stripe virus (LYSV), or two viruses, leek yellow stripe virus (LYSV) and onion yellow dwarf virus (OYDV), and the strains of the species native to Hokkaido were infected with four viruses, garlic viruses (GarVs), leek yellow stripe virus (LYSV), onion yellow dwarf virus (OYDV), and garlic latent virus (GLV).

### 1. Sterilization of the material

Scaly bulbs of a garlic were decomposed into cloves and the outer coat was removed. Then after washing with benzalkonium chloride and water, the base of the clove was cut into sections of a 1 cm cube. The sections were immersed in 70% ethanol for 5 minutes, followed by washing with sterilized water.

### 2. Preparation of explants

After sterilization of the material, the remaining storage leaf portion was removed to expose the foliage leaf. The upper part of the foliage leaf was cut off so that the height of the foliage leaf was about 0.5 cm. After vertically splitting into four parts, the remaining lower part of the foliage leaf was peeled off, and the shoot apex was removed. The remaining base was cut into a section of about 2 mm in thickness to prepare an explant.

### 3. Medium

The Linsmaier and Skoog medium (hereinafter referred to as the LS medium) to which no plant hormones were added was used for culture.

### 4. Culturing

The prepared explant was implanted onto the LS medium, and subjected to a stationary culture at 25°C under illumination for 16 hours per day.

### 5. The formation of domy tissues and isolated culture

One week after the start of culturing, domy tissues of about 0.5 mm in diameter were formed on some of the explants. Twenty to thirty domy tissues were formed per scale. The domy tissues were cut out with a scalpel, and implanted to the LS medium to which no hormones were added, and then subjected to a stationary culture at 25°C under illumination for 16 hours per day. The domy tissues turned green and grew into plants in 2-4 weeks. About 100% of the isolated domy tissues grew in this manner. The plants were implanted to a new culture medium, and on continued culture the plants became rooted. The rooted plants were implanted to an earth-filled polypot and cultivated.

### 6. virus testing

The virus testing was performed using as test specimen the leaf blade of garlic obtained by isolated culture of the domy tissue. The virus testing was performed by RT-PCR using part of the viral gene sequence as the primer.

RNA was extracted from the leaf blade, from which cDNA was synthesized using a cDNA synthesis kit. With the cDNA as the template, PCR was performed using primers for viral detection. The primers had been designed based on the base sequence of the gene of a virus that infects garlic so as to obtain amplification products specific to the virus. Specific primers were designed for each of the four viruses, garlic viruses (GarVs), leek yellow stripe virus (LYSV), onion yellow dwarf virus (OYDV), and garlic latent virus (GLV), and a PCR reaction was performed. After the PCR reaction, the presence of specific amplification products was investigated by agarose gel electrophoresis to determine the presence of the virus.

As a result, in any of the Fukuchi white species and the Hokkaido native species, no amplification products that indicate the presence of the virus were confirmed from the plants that were obtained by the isolated culture of the domy tissues. Amplification products that indicate the presence of the virus were confirmed in the plants obtained by a continued culture without isolating the material garlic and the domy tissues. Thus, it was confirmed that virus-free garlic can be obtained by isolated culture of the domy tissue formed in the culture of the foliage leaf base.

The primers used for viral detection were as follows:

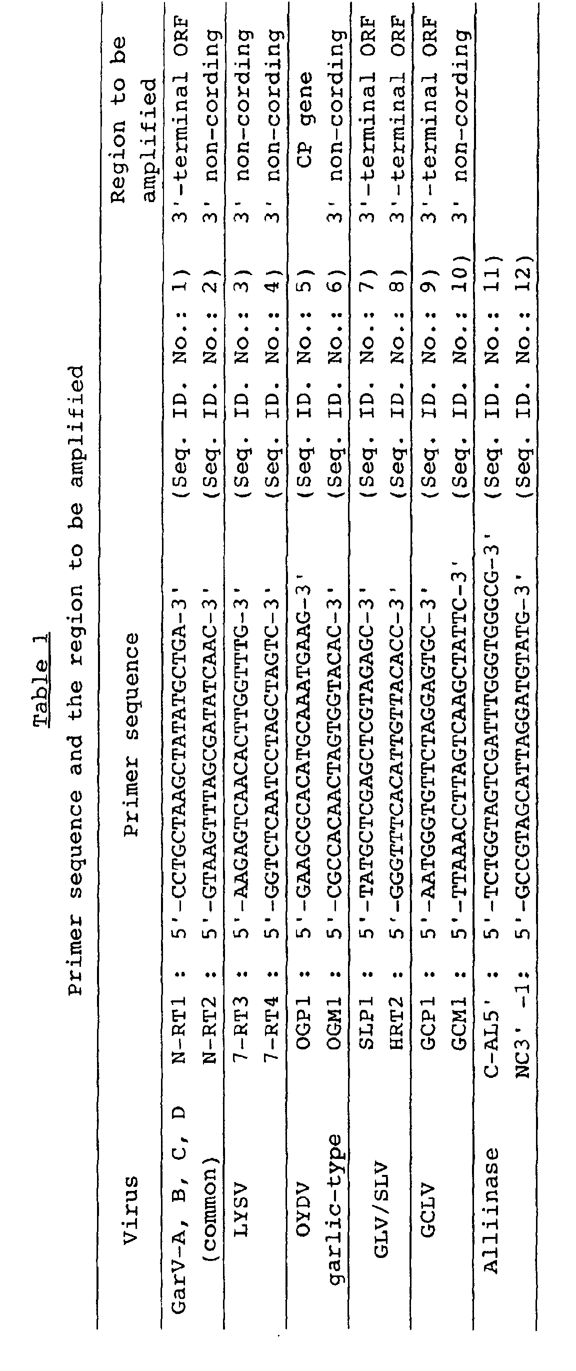

The result of virus testing is also shown below:

**Table 2**

| Result of virus testing for each plant | | | | |
|---|---|---|---|---|
| Plant | Virus | | | |
| | GarVs | LYSV | OYDV | GLV |
| Fukuchi white species (one virus species) | | | | |
| (1) Parent plant | - | + | - | - |
| (2) Regenerated plant without isolating the domy tissue | - | + | - | - |
| (3) Regenerated plant by isolating the domy tissue (a total of 11 plants) | - | - | - | - |
| Fukuchi white species (two virus species) | | | | |
| (1) Parent plant | - | + | + | - |
| (2) Regenerated plant without isolating the domy tissue | - | + | + | - |
| (3) Regenerated plant by isolating the domy tissue (a total of 5 plants) | - | - | - | - |
| Hokkaido native species | | | | |
| (1) Parent plant | + | + | + | + |
| (2) Regenerated plant without isolating the domy tissue | + | + | + | + |
| (3) Regenerated plant by isolating the domy tissue (a total of 6 plants) | - | - | - | - |

### Industrial Applicability

In accordance with the culture method of the present invention for generating virus-free plants, a larger amount of virus-free plants can be obtained as compared to the shoot apex culture method. In the conventional shoot apex culture method, it was common that there are only one or a few shoot apex per scale and one shoot apex produces only one plant, and thus it was difficult to obtain a large quantity of virus-free plants. In contrast, the method of culturing the foliage leaf base produces a plurality of plants: in the case of garlic, one scale produces 20-30 domy tissues. Therefore, a large scale propagation of virus-free plants is facilitated.

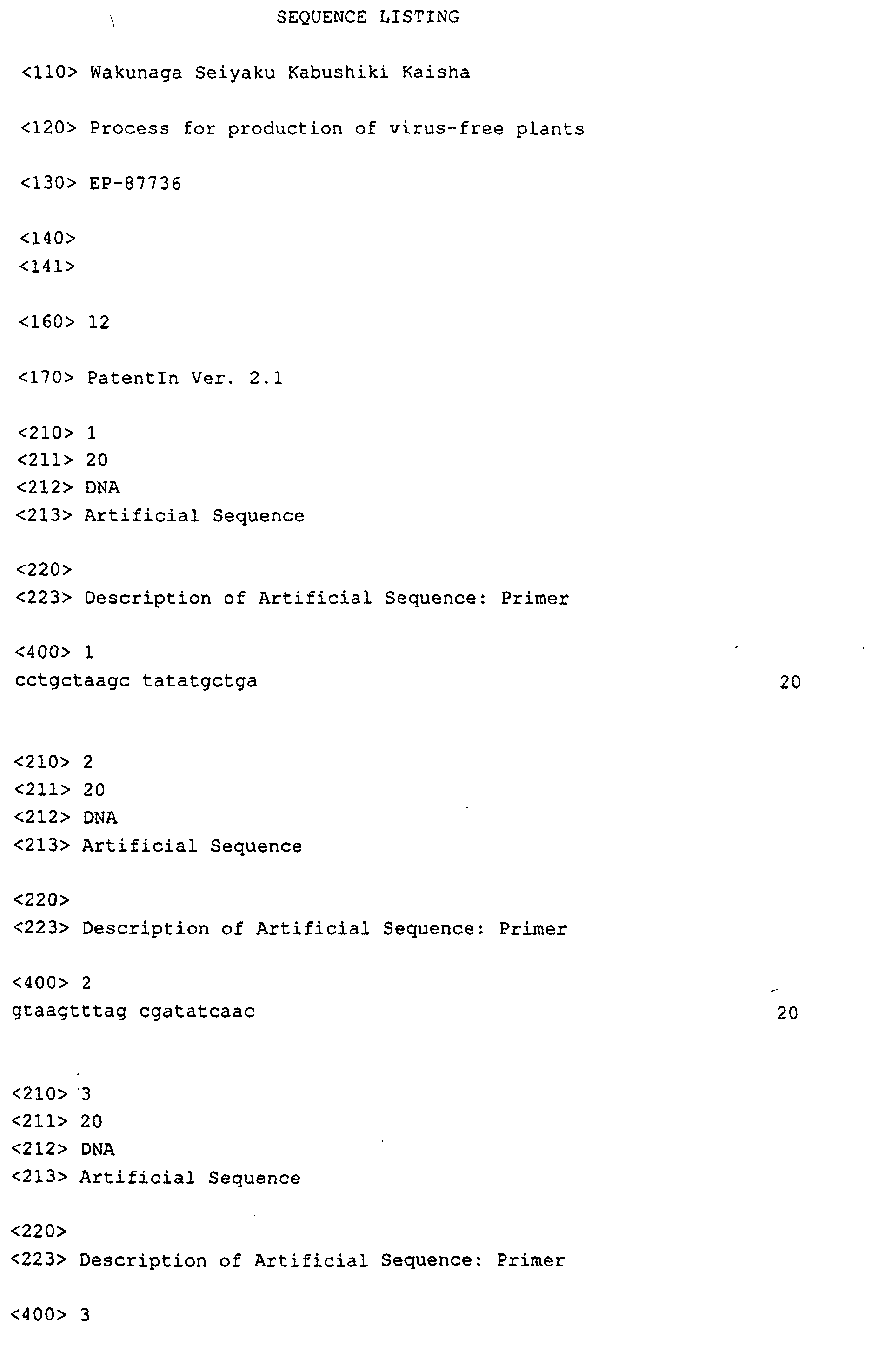

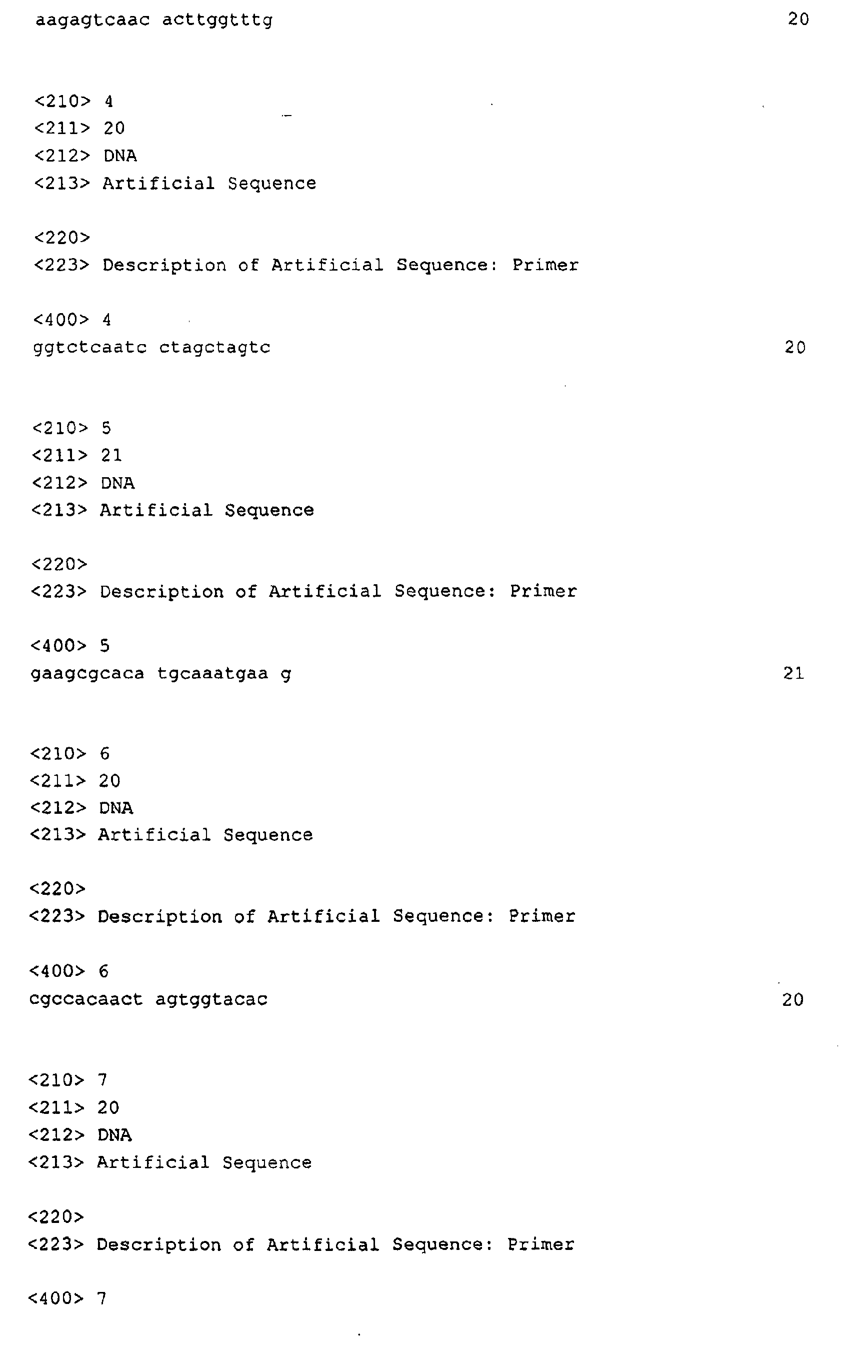

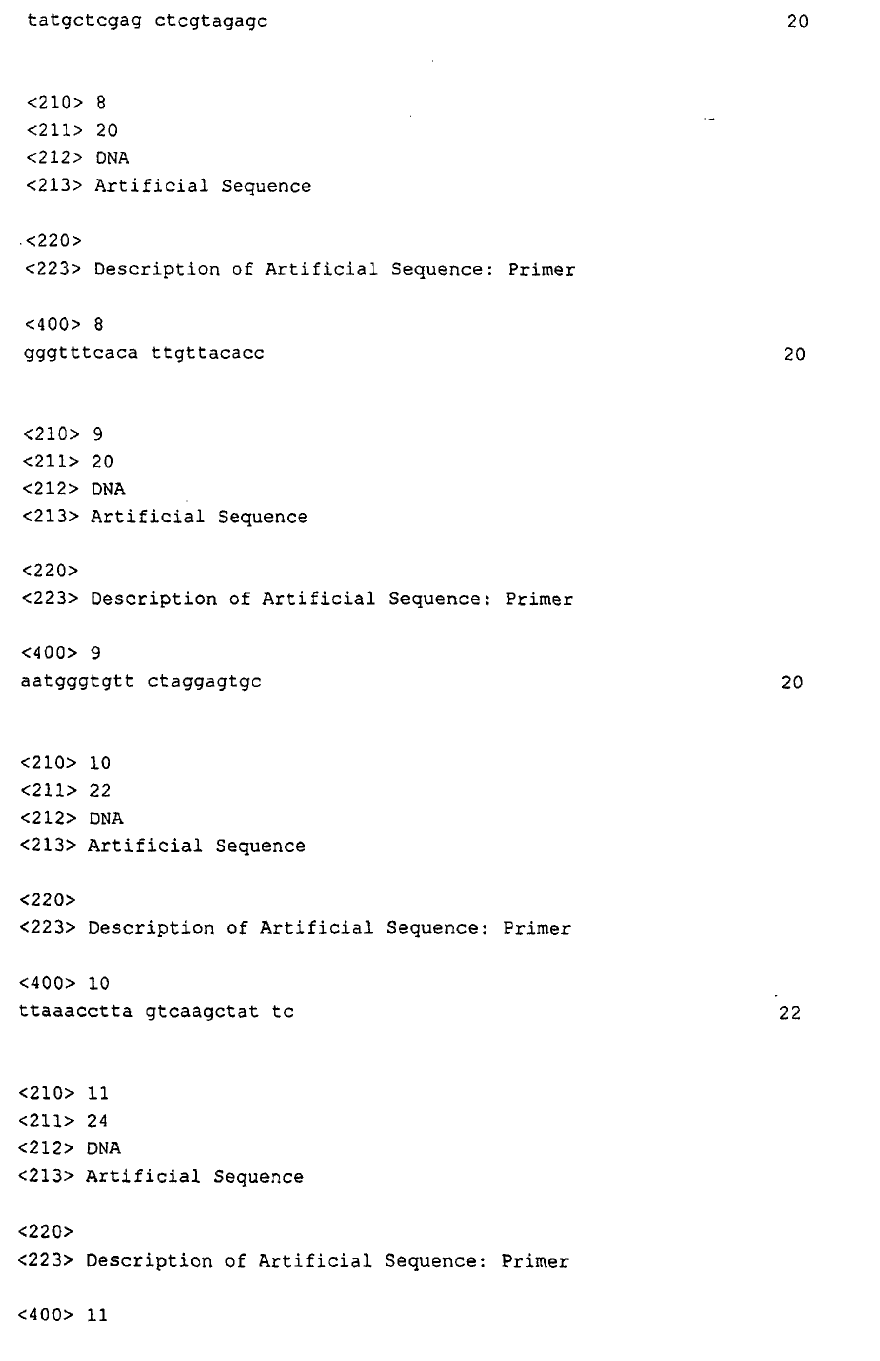

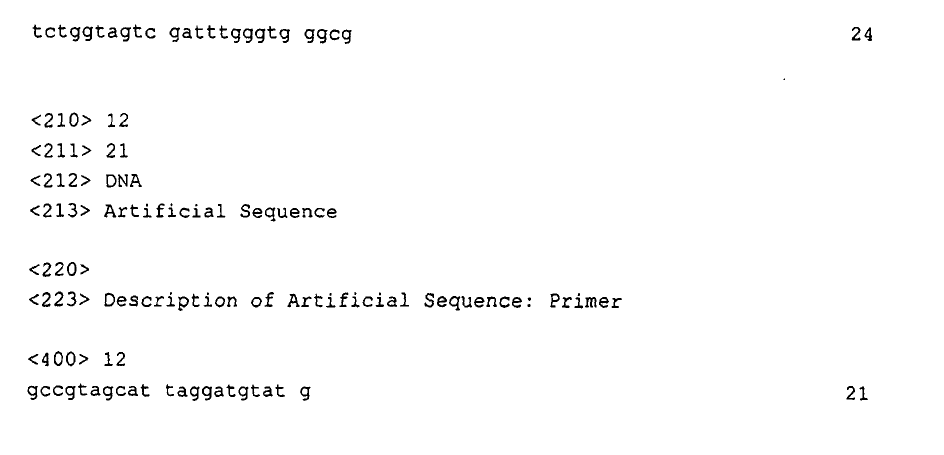

## Claims

1. A method of generating virus-free plants **characterized in that** a domy tissue formed by culturing the foliage leaf base of a plant that propagates via scaly bulbs or bulbs is isolated and cultured.

2. The method according to claim 1 wherein an explant comprising the foliage leaf base from which the shoot apex and foliage leaves have been removed is cultured in the absence of plant hormones to form a domy tissue.

3. The method according to claim 1 or 2 wherein said foliage leaf base is a section from the joint to a part 1-3 mm lower therefrom of a foliage leaf.

4. The method according to any one of claims 1 to 3 wherein the plant that propagates with scaly bulbs or bulbs is a plant of the genus *Allium.*

5. The method according to claim 4 wherein the *Allium* plant is *Allium sativum.*
